(19) Europäisches Patentamt
European Patent Office
Office européen des brevets

(11) Publication number: 0 049 593 B1

(12) EUROPEAN PATENT SPECIFICATION

(45) Date of publication of patent specification: 27.08.86

(51) Int. Cl.⁴: C 07 D 215/56, A 61 K 31/47

(21) Application number: 81304481.5

(22) Date of filing: 29.09.81

# (54) 1-Ethyl-6-fluoro-1,4-dihydro-4-oxo-7-(1-piperazinyl)-3-quinoline carboxylic acid and metal salts thereof useful in burn therapy.

(30) Priority: 02.10.80 US 193307

(43) Date of publication of application:
14.04.82 Bulletin 82/15

(45) Publication of the grant of the patent:
27.08.86 Bulletin 86/35

(84) Designated Contracting States:
AT BE CH DE FR GB IT LI LU NL SE

(58) References cited:
GB-A-1 574 285

(73) Proprietor: Fox, Charles I., Jr.
3410 Galt Ocean Drive
Fort Lauderdale, Florida 33308 (US)

(73) Proprietor: Modak, Shanta M.
184 Howland Avenue
River Edge New Jersey 07661 (US)

(72) Inventor: Fox, Charles I., Jr.
3410 Galt Ocean Drive
Fort Lauderdale, Florida 33308 (US)
Inventor: Modak, Shanta M.
184 Howland Avenue
River Edge New Jersey 07661 (US)

(74) Representative: Logan, Allan Beattie
33, Audley Road
Ealing London W5 3ES (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 049 593 B1

Courier Press, Leamington Spa, England.

**Description**

Background of the Invention

Despite the development of effective topical and systemic antibiotics, invasive wound sepsis and septicemia from pseudomonas aeruginosa remain a problem in seriously burned patients. Emergence and development of drug resistance species of bacteria have defied the control obtained through the regimen of potent antibiotics. In recent years numerous reports of gentamicin resistant gram negative organisms (Shulman, J.A., Terry, P. M. Hough, C.E.: Colonization with a gentamicin resistant pseudomonas aeruginosa pyocine type 5 in a burn unit. *J. of Inf. Diseases 124*:S18, 1971), especially pseudomonas, have appeared in the literature. (Snelling, C.F.T., Ronald, A.R., Cates, C.Y., et al.; Resistance of gram negative bacilli to gentamicin *J. of Inf. Diseases 124*:S264, 1971; Chadwick, P: Resistance of pseudomonas aeruginosa to gentamicin. *Canadian Med. Assoc. J. 109*:585, 1973; Bryan, L.E., Shahrabadi, M.S., Van Denelzen, H.M.: Gentamicin resistance in pseudomonas aeruginosa. R-factor mediated resistance. *Antimicrobial Agents and Chemotherapy 6*:191, 1974). Although silver sulfadiazine (AgSD), the most promising topical agent in the treatment of burn wound infections in this decade (Fox, Jr., C.L.: A new topical therapy for pseudomonas in burns. *Arch. Surg. 96*:184, 1968; Fox, Jr., C. L. Rappole, B. W., Stanford, J.W.: Control of pseudomonas infection in burns by silver sulfadiazine. *Sug. Gyn. Obstr. 128*:1021, 1969), appeared to surmount these problems, pseudomonas infections resistant to silver sulfadiazine treatment have been reported recently in burned patients (Gayle, W.E., Mayhall, C.G., Lamb, A., et al: Resistant enterobacter cloacal in a burn center. The ineffectiveness of silver sulfadiazine. *J. of Trauma 18*:327, 1978; Heggers, J.P., Robson, M.C.: The emergence of silver sulfadiazine resistant pseudomonas aeruginosa. *Burns 5*:184, 1978).

Similar occurrence of occasional AgSD resistant pseudomonas infections in patients have been observed in other parts of the world. Several such resistant strains have been obtained and the nature of their resistance studied in an experimental burn model. This investigation revealed an unusual phenomena, namely, normal sensitivity of pseudomonas to AgSD *in vitro*, but resistance to topical AgSD therapy in infected burn wounds in mice and rats. (Modak, S., Stanford, J.W., Bradshaw W., Fox, Jr., C.L.: Silver sulfadiazine resistant pseudomonas infection in experimental burn wounds. 3rd Intrl. Congr. of Pharma. Treatment of Burns, 1980 (in press) ed. Donati L., Burke, J., Berteilli, A., Italy.)

Comparative studies of the virulence and drug sensitivity of *in vivo* AgSD sensitive and nonsensitive strains were carried out to investigate the possible mechanism of *in vivo* resistance. Since all the resistant strains obtained from burn patients appeared to be sensitive *in vitro*, the evaluation of a topical agent for its effectiveness was determined in experimental burn models. Several other antibacterial agents known to be effective *in vitro* were also ineffective against these strains.

The continued search for an effective topical agent has led to the discovery that a synthetic analogue of nalidixic acid, 1-ethyl-6-fluoro-1,4-dihydro-4-oxo-7-(1-piperazinyl)quinolinecarboxylic acid possessing high antipseudomonas activity *in vitro* (Ito, A., Hira, K., Inoue, M., et al: In vitro antibacterial activity of AM-715, a new nalidixic acid analog. *Antimicrobial Agents and Chemotherapy* 17:103, 1980, and French patents 879,106 and 870,576) are effective in controlling AgSD resistant pseudomonas infections in burned mice. It has also been found that metal salts of 1-ethyl-6-fluoro-1, 4-dihydro-4-oxo-7-(1-piperazinyl)-3-quinoline carboxylic acid wherein the metal moiety is selected from the group consisting of silver, zinc, cerium and cobalt are useful in the treatment of burns in animal and man.

It has been disclosed in British Patent 1,574,285 that 1-ethyl-6-fluoro-1,4-dihydro-7-(1-piperazinyl)-4-oxo-quinoline-3-carboxylic acid and salts and hydrates thereof possess anti-bacterial activity and are useful in chemo-therapy, but there is no disclosure of use for treating burns in animal or man.

Summary of the Invention

In accordance with one aspect of the present invention there is provided a metal salt of 1-ethyl-6-fluoro-1,4-dihdyro-4-oxo-7-(1-piperazinyl)-3-quinoline carboxylic acid wherein the metal moiety is selected from the group consisting of silver, zinc, cerium and cobalt.

In accordance with another aspect of the present invention there is provided a composition for treating surface infections and burns by topical application to the affected surface, which comprises a metal salt of 1-ethyl-6-fluoro-1,4-dihydro-4-oxo-7-(1-piperazinyl)-3-quinoline carboxylic acid wherein the metal moiety is selected from the group consisting of silver, zinc, cerium and cobalt.

In accordance with another aspect of the present invention there is provided the use of a composition made from an effective antibacterial amount of 1-ethyl-6-fluoro-1,4-dihydro-4-oxo-7-(1-piperazinyl)-3-quinoline carboxylic acid and a physiologically acceptable water dispersible or water soluble hydrophillic carrier for the manufacture of a medicament for treating burns in animal or man by topical application to the affected surface.

The invention also includes a method of preparing the antimicrobial composition useful for topical application in the treatment of burns and the like which comprises incorporating finely divided 1-ethyl-6-fluoro-1,4-dihydro-4-oxo-7-(1-piperazinyl)-3-quinoline carboxylic acid or a metal salt thereof selected from the group consisting of the silver salt, zinc salt, cerium salt, and the cobalt salt in a water dispersible hydrophilic carrier, the aforesaid compound or the aforesaid metal salt thereof being present in the resulting composition or admixture in an effective antimicrobial amount.

Detailed Description of the Invention

1-ethyl-6-fluoro-1,4-dihydro-4-oxo-7(1-piperazinyl)-3-quinoline carboxylic acid has the structure:

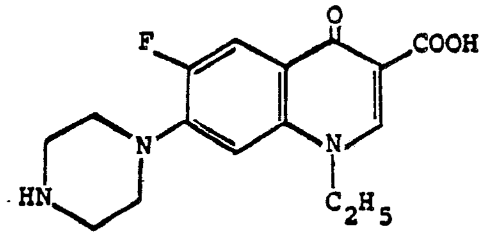

This compound is known to have high antibacterial activity *in vitro* against standard bacterial strains such as B. subtilis, S. aureus, P. aeruginosa, and E. coli strains. Ito, A. et al., *Antimicrobial Agents and Chemotherapy* *17*:103,1980, *supra*.

However, unlike several other compounds which have high *in vitro* antibacterial activity, but are ineffective in controlling silver sulfadiazine resistant pseudomonas infections in burned mice, this compound and metal salts thereof such as silver, zinc, cerium, and cobalt salts, unexpectedly are effective in controlling such infections. Other surface infections such as ulcers, bed sores and surface wounds may also be controlled.

These compounds may be applied directly to the surface or burn wounds or may be employed as a component of a composition along with a physiologically acceptable carrier. Whether employed directly or in a composition, the compounds should be applied in effective antibacterial amounts. Such amounts may vary widely depending upon the bacterial strain involved, but typically will vary from about 0.001 percent to about 10.0 percent by weight, preferably from about 0.01 percent to about 1.0 percent when the compounds are employed in compositions.

As indicated hereinabove, 1-ethyl-6-fluoro-1,4-dihydro-4-oxo-7-(1-piperazinyl)-3-quinoline carboxylic acid is a known compound which may be obtained directly or synthesized by known methods. Metal salts thereof wherein the metal moiety is silver, zinc, cerium, or cobalt, are novel compounds which are useful in burn therapy and may unexpectedly provide superior results when compared with the carboxylic acid itself. These metal salts may be prepared as follows.

The sodium salt of 1-ethyl-6-fluoro-1,4-dihydro-4-oxo-7-(1-piperazinyl)-3-quinoline carboxylic acid may be prepared by adding an equimolar amount of sodium hydroxide. Each metal salt may then be prepared by reacting the sodium salt with a stoichiometric quantity of a metal salt such as silver nitrate, cerous chloride, zinc nitrate, or the like. Of the metal salts, the silver salt has been found to be particularly effective.

When the compound of a metal salt thereof are employed in a composition with a physiologically acceptable carrier, the carrier is desirably a conventional water-dispersible, hydrophilic carrier, particularly a conventional semi-soft or cream-like, water-dispersible or water-soluble, oil-in-water emulsion, which may be applied to an affected burn surface with a minimum of patient discomfort.

Suitable compositions may be prepared by merely incorporating or homogeneously admixing finely divided compound with the hydrophilic carrier or base or ointment. One technique in accordance with this invention for incorporating the silver compound in a hydrophilic ointment, such as an oil-in-water emulsion, involves reacting equimolar aqueous solutions of silver nitrate and the sodium salt of the compound to yield a white precipitate which is the silver salt. The resulting precipitate, after washing and drying, is then mixed or blended with the candidate hydrophilic ointment, such as the oil-in-water emulsion, to yield a composition comprising the silver salt dispersed in the ointment.

Compositions in accordance with this invention containing the compound or a metal salt dispersed in a water-dispersible hydrophilic carrier or ointment, e.g., a hydrophilic oil-in-water emulsion, are usually characterized by the following components and percentages by weight set forth in accompanying Table I:

TABLE I

| Component | % By Weight |
|---|---|
| Petrolatum | 0—25 |
| Water-insoluble $C_{16}$—$C_{22}$ fatty alcohol | 7—45 |
| Emollient | 0—15 |
| Emulsifying Agents, preferably non-ioinic | 4—16 |
| Humectant | 7—40 |
| Compound or salt | 0.001—10 |
| Preservative | 0—0.3 |
| Deionized or Distilled Water q.s. | 100 |

The fatty alcohols, stearyl alcohol, cetyl alcohol, lauryl alcohol and myristyl alochol are useful in the preparation of compositions in accordance with this invention. These preferential oil-soluble fatty alcohols act as a stiffener in the resulting composition. As the emollient, isopropyl myristate, lanolin, lanolin derivatives, isopropyl palmitate, isopropyl stearate and the corresponding sebacates and other known emollients are suitable. As the emulsifying agent sorbitan monooleate, such as an amount in the range 0.5—4 percent by weight, and polyoxyl 40 stearate in an amount in the range 7—12 percent by weight, both non-ionic emulsifying agents are satifactory. A suitable humectant would be propylene glycol, sorbitol or glycerin or mixtures thereof, all being water-soluble compounds. A suitable preservative would be any of the useful conventional water-soluble preservatives which exhibit anti-microbial activity, such as sorbic acid, benzoic methylparaben, propylparaben, and mixtures thereof.

In the formulation of a composition having the make-up set forth in Table I hereinabove, as the amount of aqueous phase is increased, the solid content, i.e., the water-immiscible or water-insoluble components, e.g., fatty alcohol, such as stearyl alcohol, and/or petrolatum, must also be increased relatively to help stiffen the composition. The preservative, e.g., methylparaben, is employed in the formulation only as a preservative for the overall composition and, as indicated, methylparaben was found to be a satisfactory preservative. Methylparaben, as indicated, however, may also be used in combination with propylparaben.

Accordingly, compositions useful in the practices of this invention would include compositions comprising 0—25 percent by weight petrolatum, 7—45 percent by weight stearyl alcohol, 0—15 percent by weight isopropyl myristate, 5—20 percent by weight of an emulsifying agent, 7—40 percent by weight propylene glycol, 0.001—10 percent by weight compound or metal salt, the remainder being water, as required to bring the total percentages to 100 percent. Other compositions useful would include compositions consisting essentially of 0.01—1.0 percent by weight compound or salt, 7—8 percent by weight propylene glycol, 38—44 percent by weight water, 14—18 percent by weight petrolatum, 14—18 percent by weight stearyl alcohol, 5—8 percent by weight isopropyl myristate, 0.5—2 percent by weight sorbitan monooleate and 6—10 percent by weight polyoxyl 40 stearate. Another composition useful in the practice of this invention would include the composition consisting essentially of 0—25 percent by weight petrolatum, 7—45 percent by weight of an aliphatic fatty alcohol having a carbon atom content in the range $C_{16}$—$C_{22}$, 0—15 percent by weight of an emollient, 7—16 percent by weight of an emulsifying agent, 7—14 percent by weight of a humectant and 0.01—1.0 percent by weight of the compound or one of its metal salts.

The results of various experiments illustrating the practices of this invention are now set forth.

EXPERIMENTAL DETAILS

Methods and Materials

*Bacterial Strains*: Ps. Boston was the strain used in our previous investigations (Fox, Jr., C.L., Sampath, A.C., Stanford, J.W.: Virulence of pseudomonas infection in burned rats and mice. *Arc. Surg. 101*: 508,1970); Ps. Mangalore was isolated from a burn patient in Kasturba Medical College, Mangalore, Inida; Ps. 181 was obtained from Hopital de los Ninos, Lima, Peru; and AgSD resistant Ps. Boston was produced in our laboratory by repeatedly growing this organism in medium containing increasing amounts of AgSD.

*In vitro assay of microbial inhibition*: Inhibition indices are obtained by tube dilution tests using nutrient broth. Growth in the presence and absence of drugs was observed by turbidity measurement after

incubation at 37°C for 24—48 hours (Fox, Jr., C.L., Modak, S.M., Stanford, J.W.: Cerium sulfadiazine as a topical agent for burn wound infections: A comparison with silver sulfadiazine and zinc sulfadiazine. *Burns* *4*:233, 1978).

*Animal experiments:* Mice (female Swiss 18—22 grams) received scalds using methods reported previously (Fox, Jr., C.L.: A new topical therapy for pseudomonas in burns. *Arch. Surg. 96*:184, 1968; Fox, Jr., C.L., Sampath, A.C., Stanford, J.W.: Virulence of pseudomonas infection in burned rats and mice. *Arch. Surg. 101*:508, 1970; Fox, Jr., C.L., Modak, S.M., Stanford, J.W.: Cerium sulfadiazine as a topical agent for burn wound infections: A comparison with silver sulfadiazine and zinc sulfadiazine. *Burns 4*:233, 1978). The wounds were contaminated one hour post burn with freshly prepared 18—20 hour broth culture of pseudomonas diluted to optical density 0.30. Infection was induced by immersing the tail in the culture.

The first treatment was administered 4 hours post infection by rubbing the medicated creams over all burned surfaces. All drugs used were mixed in a cream base such as described hereinabove. Thereafter, all animals were observed and treated once daily. The primary criterion was survival. Animals that succumbed were autopsied and the cardiac blood cultured to verify the presence of pseudomonas.

Results

*In vitro tests:* The minimum inhibitory concentrations (MIC) of 1-ethyl-6-fluoro-1,4-dihydro-4-oxo-7-(1-piperazinyl)-3-quinoline carboxylic acid against various bacteria are shown in Table 2. Ps. Boston, Ps. Mangalore and Ps. 181 were sensitive to 0.05 to 0.1 µmole/ml AgSD whereas the MIC of the carboxylic acid against these organisms were as low as 0.004—0.008 µmole/ml.

In the case of E. coli, staph. aureus, klebsiella and proteus, the MIC of the carboxylic acid is about 1/20 less than that of silver sulfadiazine.

TABLE 2

Antimicrobial Spectrum of Silver Sulfadiazine and 1-Ethyl-6-fluoro-1,4-dihydro-4-oxo-7-(1-piperazinyl)-3-quinoline carboxylic acid

| | MIC (µmole/ml) | |
|---|---|---|
| Organism | AgSD | Carboxylic Acid |
| Pseudomonas Boston | 0.05 | 0.004 |
| Pseudomonas Mangalore | 0.05 | 0.008 |
| Pseudomonas 181 | 0.1 | 0.008 |
| Escherichia Coli | 0.0125 | 0.006 |
| Staphylococcus Aureus | 0.05 | 0.003 |
| Kleb. Pneumoniae | 0.05 | 0.001 |
| Proteus vulgaris | 0.003 | >0.0002 |

5 ml of nutrient broth containing the drug was inoculated with 0.2 ml of $10^{-4}$ dilution of overnight culture.
0.1 µmole AgSD corresponds to 35.7 µg
0.1 µmole carboxylic acid corresponds to 31 µg.

In as much as wound exudates contain large amounts of proteins and chlorides, it is possible that some of the topical agent may react with these molecules and only the remainder will be available for the bacteria. To evaluate the extent of the loss of the antibacterial agent due to the exudate, the MIC of AgSD and the carboxylic acid against Ps. Boston and Ps. Mangalore grown in nutrient broth containing 10% human plasma was determined. The MIC of AgSD in the medium was 0.4 µmole/ml which is 8 times more than that in nutrient broth whereas it was the same for the carboxylic acid in both the media (Table 3).

*In vivo effectiveness of various topical agents.* To investigate whether the *in vivo* drug resistance observed in some of the pseudomonas strains is specific to AgSD, the efficacy of other commonly used as well as newly synthesized topical agents was tested in burned mice using AgSD resistant Ps. Mangaloe and AgSD sensitive Ps. Boston as the infecting agents. The results are summarized in Table 4. All of the topcial agents with the exception of Furacin, Sulfamylon acetate, and gentamicin sulfadiazine protected the mice completely againsty Ps. Boston infection but none of the topical agents were effective against Ps. Mangalore infection.

*In vivo efficacy of 1-ethyl-6-fluoro-1,4-dihydro-4-oxo-7-(1-piperazinyl)-3-quinoline carboxylic acid.*

In vivo efficacy of the carboxylic acid against Ps. Mangalore, Ps. 181 and AgSD resistant Ps. Boston was tested and compared with that of AgSD in burned mice and the results are summarized in Table 5. After

infection with these resistant strains, the mortality with silver sulfadiazine therapy ranged from 80—100 percent by the 8th day post burn. In contrast, in the groups of mice receiving topical therapy with the carboxylic acid, the mortality was 0% for both Ps. Mangalore and Ps. 181 infection when the concentration of the drug in the cream was 10 mM/kg. When lower amounts were used, there was 20—40 percent mortality.

TABLE 3

Antimicrobial Activity of Silver Sulfadiazine and 1-ethyl-6-fluoro-1,4-dihydro-4-oxo-7-(1-piperazinyl)-3-quinoline carboxylic acid Against Pseudomonas Grown in Nutrient Broth Containing 10 percent Plasma

| Organism | Bacterial Dilution | MIC (μmole/ml) | |
|---|---|---|---|
| | | AgSD* | Carboxylic acid |
| Ps. Boston | $10^{-1}$ | 0.4 | 0.0125 |
| | $10^{-2}$ | 0.4 | 0.006 |
| Ps. Mangalore | $10^{-1}$ | 0.4 | 0.0125 |
| | $10^{-2}$ | 0.4 | 0.006 |

*In nutrient broth, MIC is 0.05 μ/m. See Table 2.

0.2 ml of the overnight culture of the bacteria diluted to $10^{-1}$ and $10^{-2}$ was inoculated into 5 ml of nutrient broth containing 10 percent human plasma and the drug.

TABLE 4
Topical Therapy of Burned Mice Infected with Ps. Mangalore and Ps. Boston

| Topical Agents | Concentration | % Mortality (Days P.B.) Mangalore 4 | Mangalore 7 | Boston 4 | Boston 7 |
|---|---|---|---|---|---|
| None | — | 100 | 100 | 100 | 100 |
| Silver Sulfadiazine | 30 mM | 100 | 100 | 0 | 0 |
| Silver Sulfadiazine | 60 mM | 100 | 100 | 0 | 0 |
| Silver Sulfadiazine | 120 mM | 100 | 100 | 0 | 0 |
| Zinc Sulfadiazine | 30 mM | 100 | 100 | 0 | 0 |
| Cerium Sulfadiazine | 30 mM | 100 | 100 | 0 | 0 |
| Cobalt Sulfadiazine | 30 mM | 80 | 100 | 0 | 0 |
| Cobalt Sulfadiazine | 60 mM | 80 | 100 | — | — |
| Sodium Sulfadiazine | 90 mM | 100 | 100 | 0 | 20 |
| Tetracaine Sulfadiazine | 12 mM | 100 | 100 | 40 | 40 |
| Tetracaine Sulfadiazine | 30 mM | 100 | 100 | 0 | 0 |
| Tobramycin in SILVADENE[1] | 1% | 100 | 100 | — | — |
| ETDA in SILVADENE | 1% | 100 | 100 | — | — |
| Gentamicin in SILVADENE | 0.01% | 100 | 100 | — | — |
| Gentamicin in SILVADENE | 0.001% | 80 | 100 | 0 | 10 |
| Zinc Sulfadiazine in SILVADENE | 30 mM | 90 | 100 | 0 | 0 |
| Zinc Sulfadiazine in SILVADENE | 15 mM | 100 | 100 | 0 | 0 |
| Zinc Sulfadiazine and Cerium Sulfadiazine in SILVADENE | 15 mM each | 100 | 100 | 0 | 40 |
| Sulfamylon Acetate | | 60 | 100 | 71 | 100 |
| Silver Nitrate and Cerium Sulfadiazine | 1% + 30 mM | 50 | 100 | 0 | 0 |
| Silver Nitrate | 1% | 100 | 100 | — | — |
| Chlorhexidene | 1% | 80 | 100 | 0 | — |
| Furacin | 0.2% | 90 | 100 | 80 | 90 |
| Silver Sulfadiazine in Travase | 1% | 80 | 100 | — | — |
| Silver Phosophoformate | 1.6% | 80 | 100 | — | — |
| Gentamicin Sulfadiazine | 0.1% | 100 | 100 | 55 | 91 |
| CoSD, CeSD, ZnSd, AgSd | 30 mM | 80 | 80 | — | — |

[1]Trademark for silver sulfadiazine with a cream carrier manufactured and sold by Marion Laboratories Inc., Kansas City, Missouri 64137.

TABLE 5
Topical Therapy of Burned and Infected Mice with the Carboxylic Acid

| Groups | *No. of Mice | % Mortality (Days Post Burn) 2 | 3 | 4 | 5 | 6 | 7 | 8 |
|---|---|---|---|---|---|---|---|---|
| Control | 45 | 67 | 87 | 100 | — | — | — | — |
| Rx 30 mM AgSD | 45 | 47 | 60 | 70 | 80 | 87 | 87 | 87 |
| Rx 3—6 mM carboxylic acid | 25 | 8 | 8 | 12 | 12 | 24 | 24 | 32 |
| Rx 10 mM carboxylic acid | 22 | 0 | 0 | 0 | 0 | 0 | 0 | 0 |
| Rx 30 mM carboxylic acid | 10 | 0 | 0 | 0 | 0 | 0 | 0 | 0 |

*This is a summary of 3 groups of burned mice infected with different silver sulfadiazine resistant pseudomonas strains, viz: Ps. Mangalore, Ps. 181 and AgSD resistant Ps. Boston.

*In Vitro and In Vivo Efficacy of the Carboxylic Acid and its Silver Salt*
*In Vitro Assay:*
Minimum inhibitory concentration of AgSD, carboxylic acid, and the silver salt thereof, against various organisms.

| Organism | Drug (μmole/ml) AgSd | carboxylic acid | silver salt of carboxylic acid |
|---|---|---|---|
| Ps. Boston | .05 | 0.004 | 0.003 |
| Ps. Mangalore | .05 | 0.008 | 0.003 |
| E. Coli | 0.0125 | (less than 0.006) | (less than 0.004) |
| Hemolytic Staph. | .05 | 0.003 | 0.003 |
| Klebsiella | .05 | 0.001 | (less than 0.0008) |

Mol. wt. of carboxylic acid = 309
Mol. wt. of silver salt of carboxylic acid = 415

5 ml of nutrient broth containing different concentrations of the drugs was inoculated with 0.2 ml of $10^{-4}$ dilution of the overnight culture. The cultures were incubated for 24 hours and the growth measured by turbidity.

*In Vivo Efficacy:*

Mice anesthetized with ether were given a 30% scald by dipping the lwoer third of their body into a water bath at 67°C for 7 seconds. One hour past burn mice were given 1 ml of Normosol by I.P. injection and then infected by dipping the tail in an overnight culture of pseudomonas diluted to 0.30 O.D. at 600 nm.

The animals were divided at random into groups of 5 and topical therapy was initiated 4 hours post infection. Thereafter they were treated once daily. The primary criterian was survival. Animals that succumbed were autopsied and their cardiac blood was cultured to verify the presence of pseudomonas sepsis.

| Ps. Boston Infection: | | | Days Post Burn | | | |
|---|---|---|---|---|---|---|
| | | | 2 | 3 | 5 | 6 |
| Drug | Cage | No. | Dead (%) | | | |
| Control | 1 | 5 | 4 (80) | 1 (100) | – (100) | – (100) |
| Topical AgSD | 2 | 5 | 3 (60) | 0 (60) | 1 (80) | 0 (80) |
| 30mM /kg carboxylic acid | 3 | 5 | 0 (0) | 0 (0) | 0 (0) | 0 (0) |
| 30mM/kg silver salt of carboxylic acid | 4 | 5 | 0 (0) | 0 (0) | 0 (0) | 0 (0) |

| Ps. Boston and Ps. Mangalore Infection | | | Days Post Burn | | | | |
|---|---|---|---|---|---|---|---|
| | | | 2 | 3 | 5 | 6 | 7 |
| Drug | Cage | No. | Dead (%) | | | | |
| Ps. Boston Control | A | 5 | 5 (100) | – (100) | – (100) | – (100) | |
| Topical AgSD | B | 5 | 1+1 (40) | 1 (60) | 0 (60) | 0 (60) | |
| Ps. Mangalore Control | C | 4 | 4 (100) | – (100) | – (100) | 0 (100) | |
| Topical AgSD | D | 4 | 1 (25) | 0 (25) | 1 (50) | 1 (75) | |
| 5% Sulfamylon in Marion Base[2] | E | 4 | 2+2 (100) | – (100) | – (100) | – (100) | |
| 1% Sulfamylon in Silvadene | F | 4 | 1 (25) | 0 (25) | 1 (50) | 0 (50) | |
| 30mM /kg carboxylic acid | G | 5 | 0 (0) | 0 (0) | 0 (0) | 0 (0) | |
| 30mM /kg silver salt of carboxylic acid | H | 5 | 0 (0) | 0 (0) | 0 (0) | 0 (0) | |
| 6mM /kg salt of carboxylic acid | I | 4 | 0 (0) | 0 (0) | 0 (0) | 0 (0) | 1 (25) |

[2] Cream used for Silvadene, see footnote 1, *supra*.

**Claims for the Contracting States: BE CH DE FR GB IT LI LU NL SE**

1. A metal salt of 1-ethyl-6-fluoro-1, 4-dihydro-4-oxo-7-(1-piperazinyl)-3-quinoline carboxylic acid wherein the metal moiety is selected from the group consisting of silver, zinc, cerium, and cobalt.

2. A metal salt in accordance with Claim 1 wherein the metal moiety is silver.

3. A composition for treating surface infections and burns by topical application to the affected surface, which comprises a metal salt of 1-ethyl-6-fluoro-1, 4-dihydro-4-oxo-7-(1-piperazinyl)-3-quinoline carboxylic acid wherein the metal moiety is selected from the group consisting of silver, zinc, cerium and cobalt.

4. A composition according to Claim 3 wherein the metal moiety is silver.

5. A compsoition according to Claim 3 or 4 comprising an effective antibacterial amount of said salt and a physiologically acceptable carrier.

6. A composition according to Claim 5 wherein said carrier comprises a water dispersible or water soluble hydrophylic carrier.

7. A composition according to Claim 6, wherein said carrier comprises a semi-soft or cream like water dispersible hydrophilic oil-in-water emulsion.

8. Use of a composition made from an effective antibacterial amount of 1-ethyl-6-fluoro-1, 4-dihydro-4-oxo-7-(1-piperazinyl)-3-quinoline carboxylic acid and a physiologically acceptable water dispersible or water soluble hydrophilic carrier for the manufacture of a medicament for treating burns in animal or man by topical application to the affected surface.

9. A composition according to Claim 8 in which said carrier comprises a semi-soft or cream like water dispersible hydrophilic oil-in-water emulsion.

10. A composition according to Claim 8 or 9, in which said acid comprises from 0.001 percent to 10 per cent by weight of the composition.

**Claims for the Contracting State: AT**

1. A method of preparing the antimicrobial composition useful for topical application in the treatment of burns which comprises incorporating finely divided 1-ethyl-6-fluoro-1, 4-dihydro-4-oxo-7-(1-piperazinyl)-3-quinoline carboxylic acid or a metal salt thereof is selected from the group consisting of the silver salt, zinc salt, cerium salt, and the cobalt salt in a water dispersible hydrophylic carrier, the aforesaid compound or the aforesaid metal salt thereof being present in the resulting composition or admixture in an effective antimicrobial amount.

2. A method in accordance with Claim 1, wherein said compound or salt thereof is present in said composition in an amount in the range 0.001 to 10 per cent by weight.

3. A method in accordance with Claim 1, wherein said carrier is an oil-in-water emulsion.

4. A method in accordance with Claim 1, wherein said metal salt is prepared by reacting with the sodium salt of said compound with a stoichiometric quantity of a water soluble silver salt, cerium salt, zinc salt, or cobalt salt, so as to produce the corresponding said metal salt of said compound, followed by recovering, washing and drying the resulting metal salt of said compound.

5. A method according to any of Claims 1 to 4 wherein said composition contains both said compound and one or more of said metal salts.

6. A method of preparing a metal salt of 1-ethyl-6-fluoro-1,4-dihydro-4-oxo-7-(1-piperazinyl)-3-quinoline carboxylic acid wherein the metal moiety is selected from the group consisting of silver, zinc, cerium, and cobalt, which method comprises reacting the sodium salt of the said acid with a stoichiometric quantity of a water soluble silver salt, cerium salt, zinc salt, or cobalt salt so as to produce the corresponding said metal salt of said acid, followed by recovering, washing and drying the resulting metal salt of said acid.

**Patentansprüche für die Vertragsstaaten: BE CH DE FR GB IT LI LU NL SE**

1. Ein Metallsalz der 1-Äthyl-6-fluor-1,4-dihydro-4-oxo-7-(1-piperazinyl)-chinolin-3-carbonsäure, bei dem der Metallanteil aus der aus Silber, Zink, Cer und Kobalt bestehenden Gruppe ausgewählt ist.

2. Ein Metallsalz nach Anspruch 1, bei dem der Metallanteil Silber ist.

3. Eine Zusammensetzung zur Behandlung von Oberflächeninfektionen und Verbrennungen durch örtliche Anwendung an der betroffenen Stelle, die ein Metallsalz der 1-Äthyl-6-fluor-1,4-dihydro-4-oxo-7-(1-piperazinyl)-chinolin-3-carbonsäure umfaßt, wobei der Metallanteil aus der aus Silber, Zink, Cer und Kobalt bestehenden Gruppe ausgewählt ist.

4. Eine Zusammensetzung nach Anspruch 3, bei der der Metallanteil Silber ist.

5. Eine Zusammensetzung nach Anspruch 3 oder 4, die eine wirksame antibakterielle Menge des genannten Salzes und einen physiologisch akzeptablen Träger umfaßt.

6. Zusammensetzung nach Anspruch 5, bei der der genannte Träger einen in Wasser dispergierbarer oder in Wasser löslichen hydrophilen Träger umfaßt.

7. Eine Zusammensetzung nach Anspruch 6, bei der der genannte Träger eine halbweiche oder cremeartige, in Wasser dispergierbare, hydrophile Öl-in-Wasser-Emulsion umfaßt.

8. Verwendung einer Zusammensetzung, hergestellt aus einer wirksamen antibakteriellen Menge von 1-Äthyl-6-fluor-1,4-dihydro-4-oxo-7-(1-piperazinyl)-chinolin-3-carbonsäure und einem physiologisch akzeptablen, in Wasser dispergierbaren oder in Wasser löslichen hydrophilen Träger für die Herstellung eines Medikaments zur Behandlung von Verbrennungen bei Tieren oder Menschen durch örtliche Anwendung an der betroffenen Oberfläche.

9. Eine Zusammensetzung nach Anspruch 8, bei der der genannte Träger eine halbweiche oder cremeartige, in Wasser dispergierbare, hydrophile Öl-in-Wasser-Emulsion enthält.

10. Eine Zusammensetzung nach Anspruch 8 oder 9, bei der die genannte Säure zwischen 0,001 bis 10 Gew.-% der Zusammensetzung ausmacht.

**Patentansprüche für den Vertragsstaat: AT**

1. Verfahren zur Herstellung der antimikrobischen, für die örtliche Anwendung bei der Behandlung von Verbrennungen nützliche Zusammensetzung, umfassend die Einarbeitung von fein verteilter 1-Äthyl-6-fluor-1,4-dihydro-4-oxo-7-(1-piperazinyl)-chinolin-3-carbonsäure oder einem Metallsalz derselben, das aus

der aus dem Silbersalz, dem Zinksalz, dem Cersalz oder dem Kobaltsalz bestehenden Gruppe ausgewählt ist, in einen in Wasser dispergierbaren hydrophilen Träger, wobei die genannte Verbindung oder deren genanntes Metallsalz in der resultierenden Zusammensetzung oder Mischung in einer wirksamen antimikrobischen Menge vorhanden ist.

2. Ein Verfahren nach Anspruch 1, bei dem die genannte Verbindung oder deren Salz in der genannten Zusammensetzung in einer Menge im Bereich von 0,001 bis 10 Gew.-% vorhanden ist.

3. Ein Verfahren nach Anspruch 1, bei dem der genannte Träger eine Öl-in-Wasser-Emulsion ist.

4. Ein Verfahren nach Anspruch 1, bei dem das genannte Metallsalz hergestellt wird durch Umsetzung des Natriumsalzes der genannten Verbindung mit einer stöchiometrischen Menge eines wasserlöslichen Silbersalzes, Cersalzes, Zinksalzes oder Kobaltsalzes, um das entsprechendes Metallsalz der genannten Verbindung zu bilden worauf ein Abtrennen, Waschen und Trocknen des resultierenden Metallsalzes der genannten Verbindung folgt.

5. Ein Verfahren nach einem der Ansprüche 1 bis 4, bei dem die genannte Zusammensetzung sowohl die genannte Verbindungs als auch eines oder mehrere genannten Metallsalze enthält.

6. Ein Verfahren zum Herstellen eines Metallsalzes der 1-Äthyl-6-fluor-1,4-dihydro-4-oxo-7-(1-piperazinyl)-chinolin-3-carbonsäure, bei dem der Metallanteil aus der aus Silber, Zink, Cer und Kobalt bestehenden Gruppe ausgewählt ist, wobei das Verfahren die Umsetzung des Natriumsalzes der genannten Säure mit einer stöchiometrischen Menge eines wasserlöslichen Silbersalzes, Cersalzes, Zinksalzes oder Kobaltsalzes umfaßt, um das entsprechende genannte Metallsalz der genannten Säure zu erzeugen, worauf ein Abtrennen, Waschen und Trocknen des resultierenden Metallsalzes der genannten Säure folgt.

**Revendications pour les Etats contractants: BE CH DE FR GB IT LI LU NL SE**

1. Sel métallique d'acide 1-éthyl-6-fluoro-1,4-dihydro-4-oxo-7-(1-pipérazinyl)-3-quinoléine-carboxylique, dans lequel le fragment métallique est choisi dans le groupe comprenant l'argent, le zinc, le cérium et le cobalt.

2. Sel métallique selon la revendication 1, dans lequel le fragment métallique est l'argent.

3. Composition de traitement des infections et brûlures superficielles par application locale à la surface attaquée, qui comprend un sel métallique d'acide 1-éthyl-6-fluoro-1,4-dihydro-4-oxo-7-(1-pipérazinyl)-3-quinoléine-carboxylique dans lequel le fragment métallique est choisi dans le groupe comprenant l'argent, le zinc, le cérium et le cobalt.

4. Composition selon la revendication 3, dans laquelle le fragment métallique est l'argent.

5. Composition selon la revendication 3 ou 4, comprenant une proportion anti-bactérienne efficace dudit sel et un véhicule physiologiquement acceptable.

6. Composition selon la revendication 5, dans laquelle ledit véhicule comprend un véhicule hydrophile dispersable dans l'eau ou soluble dans l'eau.

7. Composition selon la revendication 6, dans laquelle ledit véhicule comprend une émulsion hydrophile huile-dans-eau semi molle ou crémeuse dispersable dans l'eau.

8. Utilisation d'une composition préparée à partir d'une proportion anti-bactérienne efficace d'acide 1-éthyl-6-fluoro-1,4-dihydro-4-oxo-7-(1-pipérazinyl)-3-quinoléine-carboxylique et un véhicule hydrophile physiologiquement acceptable dispersable dans l'eau ou soluble dans l'eau, pour la fabrication d'un médicament de traitment de brûlure chez les animaux ou l'homme par application locale sur la surface attaquée.

9. Composition selon la revendication 8, dans laquelle ledit véhicule comprend une émulsion huile-dans-eau semi molle ou crémeuse, dispersable dans l'eau.

10. Composition selon la revendication 8 ou 9, dans laquelle ledit acide comprend de 0,001 à 10% en poids de la composition.

**Revendications pour l'Etat contractant: AT**

1. Procédé de préparation d'une composition anti-microbienne convenant pour application locale lors du traitement des brûlures, qui comprend le fait d'incorporer l'acide 1-éthyl-6-fluoro-1,4-dihydro-4-oxo-7-(1-pipérazinyl)-3-quinoléine-carboxylique finement divisé ou un sel métallique de celui-ci choisi dans le groupe comprenant le sel d'argent, le sel de zinc, le sel de cérium et le sel de cobalt dans un véhicule hydrophile dispersable dans l'eau, ledit composé ou ledit sel métallique de celui-ci étant présent dans la composition ou mélange résultant en une proportion anti-microbienne efficace.

2. Procédé selon la revendication 1, dans lequel ledit composé ou ledit sel du celui-ci est présent dans ladite composition à raison de 0,001 à 10% en poids.

3. Procédé selon la revendication 1, dans lequel ledit véhicule est une émulsion huile-dans-eau.

4. Procédé selon la revendication 1, dans lequel on prépare ledit sel métallique en faisant réagir le sel de sodium dudit composé avec une proportion stoechiométrique d'un sel d'argent, sel de cérium, sel de zinc ou sel de cobalt hydrosoluble de manière à produire ledit sel métallique correspondant dudit composé puis on récupère, on lave et on sèche le sel métallique résultant dudit composé.

5. Procédé selon l'une des revendications 1 à 4, dans lequel ladite composition contient à la fois ledit

composé et un ou plusieurs desdits sels métalliques.

6. Procédé de préparation d'un sel métallique d'acide 1-éthyl-6-fluoro-1,4-dihydro-4-oxo-7-(1-pipérazinyl)-3-quinoléine-carboxylique dans lequel le fragment métallique est choisi dans le groupe comprenant l'argent, le zinc, le cérium et le cobalt, procédé qui consiste à faire réagir le sel de sodium dudit acide avec une quantité stoechiométrique d'un sel d'argent, sel de cérium, sel de zinc ou sel de cobalt soluble dans l'eau pour produire ledit sel métallique correspondant audit acide puis on récupère, on lave et on sèche le sel métallique résultant dudit acide.